(19) Europäisches Patentamt
European Patent Office
Office européen des brevets

(11) Publication number: **0 019 056**
**A1**

(12) # EUROPEAN PATENT APPLICATION

(21) Application number: 80101417.6

(22) Date of filing: 18.03.80

(51) Int Cl.³: C 07 D 221/26
//A61K31/47

(30) Priority: 26.03.79 US 23613

(43) Date of publication of application:
26.11.80 Bulletin 80/24

(84) Designated Contracting States:
BE CH DE FR GB IT NL SE

(71) Applicant: STERLING DRUG INC.
90 Park Avenue
New York New York(US)

(72) Inventor: Michalec, Stephen James
R.D. 3, Mountain View Apartments Apt. 9A
Valatie New York(US)

(74) Representative: Baillie, Iain Cameron et al,
c/o Ladas & Parry Blumenstrasse 48
D-8000 München 2(DE)

(54) **Process for preparing 11-lower alkylthio-3-oxo-lower-alkyl-hexahydro-2,6-methano-3-benzazocines.**

(57) A process for preparing a compound having the formula:

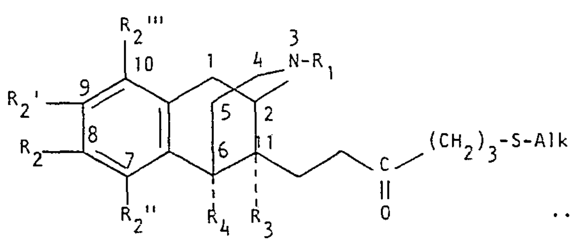

...1

where $R_1$ is hydrogen, lower-alkyl, cyclo-alkyl-lower-alkyl, phenyl-lower-alkyl, lower-alkenyl or lower alkynyl; three of $R_2$, $R_2'$, $R_2''$ and $R_2'''$ are hydrogen and the fourth is hydroxy, $R_3$ and $R_4$ are each hydrogen or lower-alkyl or $R_3$ and $R_4$ together are divalent lower-alkylene, $-(CH_2)_m$, where m is one of the integers 3 and 4; and Alk is lower-alkyl, which comprises reacting a compound having the formula:

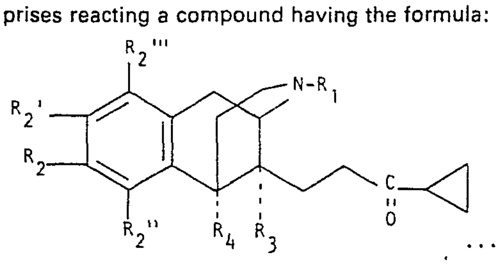

...11

where three of $R_2$, $R_2'$, $R_2''$ and $R_2'''$ are hydrogen and the fourth is lower-alkoxy, and $R_1$, $R_3$ and $R_4$ have the meanings given above, with an alkali metal lower-alkanethiol salt.

EP 0 019 056 A1

TITLE MODIFIED
see front page



"11-LOWER ALKOXY- AND LOWER-ALKYLTHIO-3-OXO-LOWER-ALKYL-HEXAHYDRO-2, 6-METHANO-3-BENZAZONCINES"

D E S C R I P T I O N

This invention relates to a novel process for preparing 3-$R_1$-7-, 8-, 9- or 10-hydroxy-11(ax)-$R_3$-6(eq)-$R_4$-11-(eq)-($CH_2CH_2COCH_2CH_2CH_2S$-Alk)-1,2,3,4,5,6-hexahydro-2,6-methano-3-benzazocines having the formula:

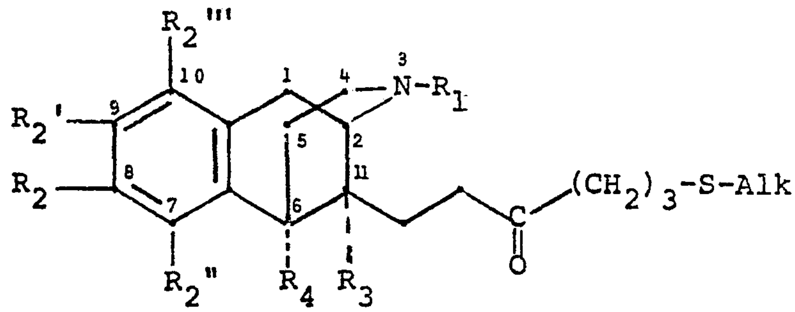

...I

where $R_1$ is hydrogen, lower-alkyl, cycloalkyl-lower-alkyl, phenyl-lower-alkyl, lower-alkenyl or lower-alkynyl; three of $R_2$, $R_2'$, $R_2''$ and $R_2'''$ are hydrogen and the fourth is hydroxy; $R_3$ and $R_4$ are each hydrogen or lower-alkyl, or $R_3$ and $R_4$ together are divalent lower-alkylene, $-(CH_2)_m-$, where m is one of the integers 3 and 4; and Alk is lower-alkyl and is an improvement over the process disclosed in Belgian Patent 864,950.

As used herein, the term lower-alkyl means saturated, acyclic groups which may be straight or branched containing from one to about seven carbon atoms as exemplified by methyl, ethyl, propyl, isopropyl, butyl, hexyl or heptyl.

As used herein, the terms lower-alkenyl and lower-alkynyl mean monovalent groups of from three to seven carbon atoms containing one double or triple bond as illustrated, for example, by 2-propenyl, 2-butenyl, 4-pentenyl, 3-methyl-2-butenyl, 1-methyl-2-propenyl, 2-methyl-2-propenyl, 2-propynyl, 2-butynyl, 4-pentynyl, 2-hexynyl and the like.

As used herein, the term cycloalkyl means saturated carbocyclic groups containing from three to seven ring carbon atoms as illustrated, for example, by cyclopropyl, cyclobutyl, cyclopentyl, cyclohexyl, 2-methylcyclobutyl, 4-ethylcyclohexyl and the like.

According to the process of the present invention, the compounds of Formula I are prepared by reacting a 3-$R_1$-7-, 8-, 9- or 10-lower-alkoxy-11(ax)-$R_3$-6(eq)-$R_4$-11(eq)-($CH_2CH_2CO$-cyclopropyl)-1,2,3,4,5,6-hexahydro-2,6-methano-3-benzazocine having the formula:

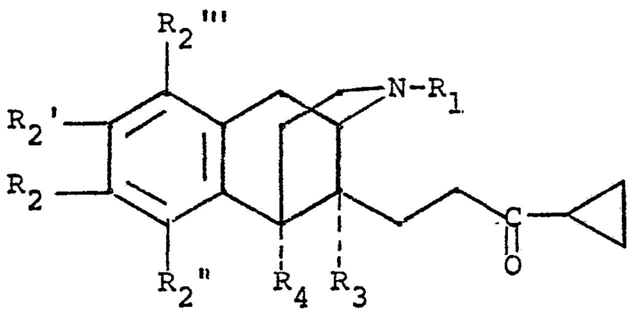

...II

where three of $R_2$, $R_2'$, $R_2''$ and $R_2'''$ are hydrogen and the fourth is lower-alkoxy containing from one to seven carbon atoms, and $R_1$, $R_3$ and $R_4$ have the meanings previously indicated, with an alkali metal salt of a lower-alkanethiol, Alk-S-M, where M is an alkali metal cation, for example lithium, sodium or potassium, and Alk has the meaning given above. The reaction is carried out in an inert organic solvent, for example toluene, xylene, dimethylformamide (DMF) or the like. The reaction can take place at ambient temperature, although the reaction rate may be slow. It is therefore preferred, in order to increase the reaction rate and thereby shorten the reaction time, to carry out the reaction at an elevated temperature, for example, up to the reflux temperature of the reaction medium.

The above described process for the preparation of the compounds of Formula I via the compounds of Formula II represents a substantial improvement over the known process for the preparation of the former as described in Belgian Patent 864,950, because the present process utilizes more readily available starting materials, i.e., the compounds of

Formula II, than the known process. The preparation of the starting materials of both the present process and the process of Belgian Patent 864,950 requires acylation of a lower-alkyl 1,2,3,4,4a,5,10,10a-octahydro-2,5-methanobenzo[g]quinoline-3-carboxylate having the Formula III below. However, the starting materials required in the present process require acylation of the compounds of Formula III with a readily available, and relatively inexpensive, cyclopropanecarbonyl halide, whereas the starting materials for the known process require acylation with a less readily available, and more expensive, γ-lower-alkylthiobutyryl halide, Alk-S-$(CH_2)_3$CO-Hal, where Alk has the meaning given above and Hal is halogen. Moreover, the present process combines two steps of the synthetic sequence, i.e., the introduction of the alkylthio (-S-Alk) function into the 11-position side chain and the ether cleavage, into one step, whereas the prior process requires ether cleavage as a separate step. The overall yields of products are thus higher in the present process than in the known process. The overall reaction sequences of the two processes as described above are illustrated as follows:

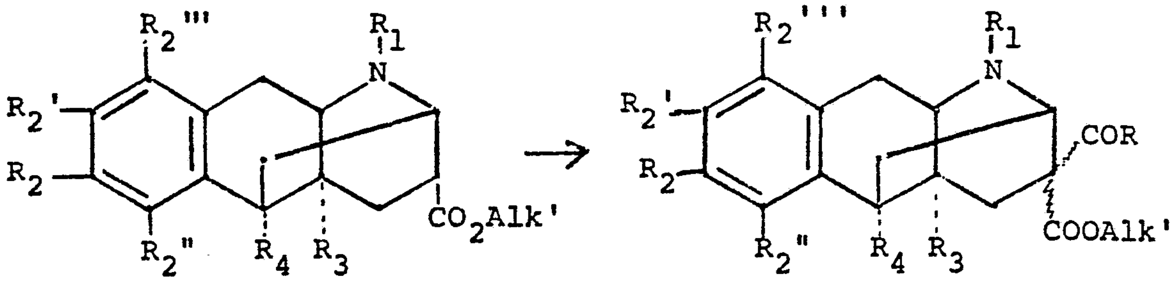

III

IV

Present process: R = cyclopropyl

Prior process: R = $(CH_2)_3S$-Alk

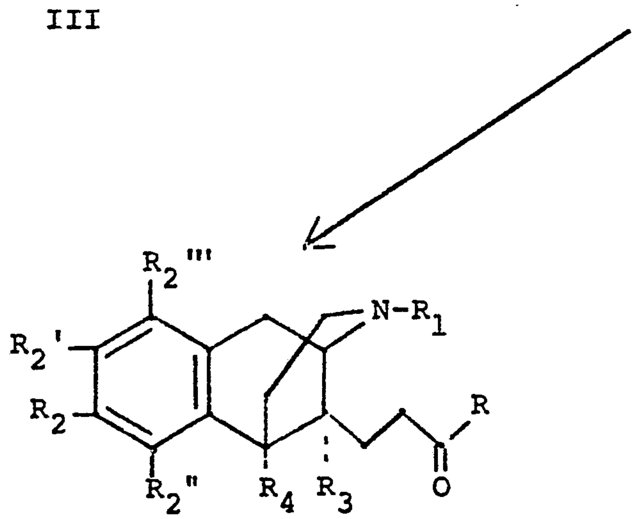

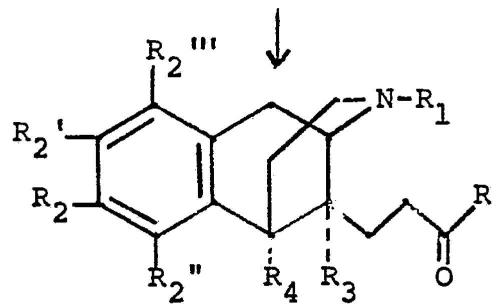

II

Prior process:
One of $R_2$, $R_2'$, $R_2''$, $R_2'''$ is lower-alkoxy; R is $(CH_2)_3S$-Alk

Present process:
One of $R_2$, $R_2'$, $R_2''$, $R_2'''$ is lower-alkoxy; R is cyclopropyl

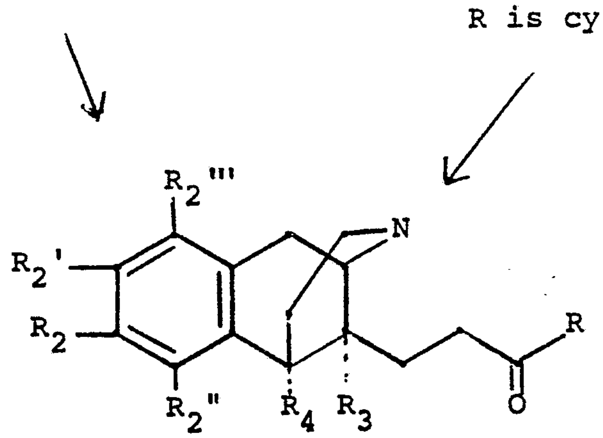

One of $R_2$, $R_2'$, $R_2''$, $R_2'''$ is hydroxy; R is $(CH_2)_3S$-Alk

As will be seen, the reaction involves ring opening of the cyclopropyl ring of the 11(eq)-($CH_2CH_2CO$-cyclopropyl) group by the lower-alkylthio anion which will also cleave any ether groups present in the molecule, i.e., the $R_2$, $R_2'$, $R_2''$ or $R_2'''$ lower-alkoxy group. Thus, the reaction requires use of at least two molar equivalents of the alkali metal lower-alkanethiol salt in order to achieve complete conversion of the starting materials of Formula II to the compounds of Formula I.

The alkali metal lower-alkanethiol salt can either be prepared in situ by addition of an alkali metal hydride, an alkali metal alkyl, for example, butyl lithium, or an alkali metal amide to a solution containing the starting material of Formula II and at least two molar equivalents of the desired lower-alkanethiol or, alternatively, the alkali metal lower-alkanethiol salt can be generated separately in the same fashion, that is by addition of an alkali metal hydride, an alkali metal alkyl or an alkali metal amide to a solution of the lower-alkanethiol in an inert organic solvent and addition of the resulting mixture to a separate solution of the starting material of Formula II in an inert solvent. In the latter case, where the alkali metal lower-alkanethiol salt is generated separately, the salt can also be prepared by addition of an alkali metal per se to a solution of the lower-alkanethiol in an inert solvent.

The compounds of Formula II and a process for their preparation, as well as the compounds of Formula I, are disclosed in U.S. Patent 4,148,794.

Due to the presence of a basic amino grouping, the free base form represented by Formula I above reacts with organic and inorganic acids to form acid-addition salts. The acid-addition salt forms are prepared from any organic or inorganic acid. They are obtained in conventional fashion, for instance either by direct mixing of the base with the acid or, when this is not appropriate, by dissolving either or both of the base and the acid separately in water or an organic solvent and mixing the two solutions, or by dissolving both the base and the acid together in a solvent. The resulting acid-addition salt is isolated by filtration, if it is insoluble in the reaction medium, or by evaporation of the reaction medium to leave the acid-addition salt as a residue. The acid moieties or anions in these salt forms are in themselves neither novel nor critical and therefore can be any acid anion or acid-like substance capable of salt formation with the base.

Representative acids for the formation of the acid-addition salts include formic acid, acetic acid, isobutyric acid, alpha-mercaptopropionic acid, trifluoroacetic acid, malic acid, fumaric acid, succinic acid, succinamic acid, tannic acid, glutamic acid, tartaric acid, oxalic acid, pyromucic acid, citric acid, lactic acid, glycolic acid, gluconic acid, saccharic acid, ascorbic acid, penicillin, benzoic acid, phthalic acid, salicylic acid, 3,5-dinitrobenzoic acid, anthranilic acid, cholic acid, 2-pyridinecarboxylic acid, pamoic acid, 3-hydroxy-2-naphthoic acid, picric acid, quinic acid, tropic acid, 3-indoleacetic acid, barbituric acid, sulfamic acid, methanesulfonic acid, ethanesulfonic acid, isethionic acid, benzenesulfonic acid, p-toluenesulfonic acid, butylarsonic acid, methanephosphonic acid, acidic resins, hydrofluoric acid, hydrochloric acid, hydrobromic acid, hydriodic acid, perchloric acid, nitric acid, sulfuric acid, phosphoric acid, arsenic acid, and the like.

The compounds of Formula I can exist in enantiomeric forms separable into enantiomers. If desired, the isolation or the production of a particular enantiomeric form can be accomplished by application of general principles known in the prior art. In the nomenclature employed for the compounds of Formula I, herein, "ax" stands for axial and "eq" for equatorial, and the configurations are given with reference to the hydroaromatic ring. Thus, the 6(eq), 11(ax) substituents of Formula I are in the <u>cis</u> configuration, whereas the 6(eq), 11(eq) substituents are in the <u>trans</u> configuration.

In standard pharmacological test procedures, the compounds of Formula I and the acid-addition salts thereof

have been found useful as depressants of the central nervous system, and more particularly have been found useful as analgesics and as antagonists of strong analgesics such as phenazocine, meperidine and morphine.

The compounds of Formula I can be administered in the same manner as knwon analgesics and antagonists of strong analgesics, i.e., parenterally or orally in any of the conventional pharmaceutical forms, as for instance solutions, suspensions, tablets, capsules, and the like.

The useful properties of the compounds of Formula I were demonstrated by standard pharmacological procedures readily carried out by technicians having ordinary skill in pharmacological test procedures, so that the actual determination of numerical biological data definitive for a particular test compound can be ascertained without the need for any extensive experimentation.

The test procedures used to determine the analgesic and analgesic antagonist activities of the compounds of Formula I have been described in detail in the prior art and are as follows: the acetylcholine-induced abdominal constriction test, which is a primary analgesic screening test designed to measure the ability of a test agent to suppress acetylcholine-induced abdominal constriction in mice, described by Collier et al., Brit. J. Pharmacol. Chemotherap. 32, 295 (1968); a modification of the anti-bradykinin test, which is also a primary analgesic screening procedure, described by Berkowitz et al., J. Pharmacol. Exptl. Therap. 177, 500-508 (1971), Blane et al., J. Pharm. Pharmacol. 19, 367-373 (1967), Botha et al., Eur. J. Pharmacol. 6, 312-321 (1969) and Deffenu et al., J. Pharm. Pharmacol 18, 135 (1966); the phenyl-p-quinone-induced writhing test, also a primary analgesic screening test, designed to measure the ability of a test agent to prevent phenyl-p-quinone-induced writhing in mice, described by Pearl and Harris, J. Pharmacol. Exptl. Therap. 154, 319-323 (1966); the rat tail flick radiant thermal heat analgesic (agonist) test described by D'Amour and Smith, J. Pharmacol. Exptl. Therap. 72, 74 (1941) as modified by Bass

and VanderBrook, J. Am. Pharm. Assoc., Sci. Ed. 41, 569 (1956); and the narcotic antagonist test using phenazocine, meperidine and morphine, which is designed to measure the ability of a test agent to antagonize the effect of phenazocine, meperidine or morphine in the above indicated rat tail flick response test, described by Harris and Pierson, J. Pharmacol. Exptl. Therap. 143, 141 (1964).

The structures of the compounds of Formula I were established by the mode of synthesis, by elementary analyses and mass, infrared and nuclear magnetic resonance spectra. The course of reactions and homogeneity of the products were ascertained by thin layer chromatography.

The manner of carrying out the process of the invention will now be described so as to enable any person skilled in the art to which it pertains to use the same. The melting points are uncorrected.

EXAMPLE 1

A mixture of 1.0 g. (0.003 mole) of 3,6(eq),11(ax)-trimethyl-8-methoxy-11(eq)-(3-cyclopropyl-3-oxopropyl)-1,2,3,4,5,6-hexahydro-2,6-methano-3-benzazocine and 0.92 g. (0.017 mole) of lithium methylsulfide in 15 ml. of dry DMF was heated under reflux under a nitrogen atmosphere for twenty-four hours and then cooled and poured into 153 ml. of 0.15 molar aqueous methanesulfonic acid. The solution was washed several times with diethyl ether, then rendered basic with ammonium hydroxide and extracted again with three portions of diethyl ether. The combined organic extracts were washed twice with water, then with brine, dried, filtered and evaporated to dryness *in vacuo* to give 0.9 g. (80%) of the crude product as orange-yellow syrup which was dissolved in ethanol, treated with a molar excess of ethanesulfonic acid and diluted with diethyl ether. The crystalline material which separated was collected and recrystallized once again from absolute ethanol/diethyl ether to give 0.3 g. of 3,6(eq),11(ax)-trimethyl-8-hydroxy-11(eq)-(6-methylmercapto-3-oxohexyl)-1,2,3,4,5,6-hexahydro-2,6-methano-3-benzazocine ethanesulfonate, m.p. 175.5-180°C.

Anal.

Calcd. for $C_{22}H_{33}NO_2S \cdot C_2H_5SO_3H$: C, 59.35; H, 8.06; N, 2.88

Found: C, 59.34; H, 8.23; N, 2.78.

Using the process of Belgian Patent 864,950, the same compound was prepared in 27% crude yield by cleaving the corresponding methyl ether with hydrogen bromide. In contrast, as indicated above, the present process, in which the methylthio function was introduced simultaneously with cleavage of the ether group, afforded an 80% crude yield for the final combined synthetic step.

EXAMPLE 2

To a mixture of 6.8 g. (0.02 mole) of 3,6(eq),11(ax)-trimethyl-8-methoxy-11(eq)-(3-cyclopropyl-3-oxopropyl)-1,2,3,4,5,6-hexahydro-2,6-methano-3-benzazocine, 100 ml. of anhydrous DMF and 4.8 g. (0.10 mole) of a 50% suspension of sodium hydride in mineral oil was added 10.8 ml. (0.10 mole) of propanethiol while maintaining a nitrogen atmosphere in the reaction vessel. When hydrogen evolution had ceased, the mixture was heated under reflux for three hours, then cooled and poured into one liter of dilute aqueous methanesulfonic acid. The aqueous solution was washed with diethyl ether, basified with ammonium hydroxide, extracted with diethyl ether, and the organic extracts were dried and evaporated to give 7.2 g. (89%) of the crude product as an oil. The latter was dissolved in absolute ethanol, and the solution was treated with a molar excess of sulfuric acid and diluted with diethyl ether. The crystalline material which separated was collected and recrystallized several times from ethanol/diethyl ether to give 1.8 g. of 3,6(eq),11(ax)-trimethyl-8-hydroxy-11(eq)-(6-propylmercapto-3-oxohexyl)-1,2,3,4,5,6-hexahydro-2,6-methano-3-benzazocine sulfate, m.p. 198-202°C.

Anal.

Calcd. for $C_{24}H_{37}NO_2S \cdot H_2SO_4$: C, 57.45; H, 7.80; N, 2.79

Found: C, 57.44; H, 7.87; N, 2.78.

BIOLOGICAL TEST RESULTS

The compounds of Formula I are generally active in the acetylcholine-induced abdominal constriction test, a

primary analgesic screening test, the anti-bradykinin test and also in the rat tail flick radiant thermal heat antagonist test.

Thus, the $ED_{50}$'s in the acetylcholine-induced abdominal constriction test and the anti-bradykinin test and the $AD_{50}$'s vs. phenazocine and morphine in the narcotic antagonist test of the compound of Example 1 were found to be, respectively, 0.1-1.0 mg./kg. (s.c.), 0.071 mg./kg. (s.c.), 0.0034 mg./kg. (s.c.) and 0.18 mg./kg. (s.c.). The same compound was found to be inactive in the tail flick agonist test at 120 mg./kg. (s.c.).

The compound of Example 2 was found to be essentially inactive in the acetylcholine-induced abdominal constriction test (60% inhibition/20 mg./kg.; 53%/10 mg./kg.; 40%/1 mg./kg.; and 7%/0.1 mg./kg., all s.c.) and also in the anti-bradykinin test [0/5 protected at 1.0 and 10.0 mg./kg. (s.c.)]. The $AD_{50}$ vs. phenazocine in the tail flick antagonist test was found to be 0.046 mg./kg. (s.c.).

CLAIMS

1. A process for preparing a compound having the Formula I (herein), where $R_1$ is hydrogen, lower-alkyl, cycloalkyl-lower-alkyl, phenyl-lower-alkyl, lower-alkenyl or lower-alkynyl; three of $R_2$, $R_2'$, $R_2''$ and $R_2'''$ are hydrogen and the fourth is hydroxy, $R_3$ and $R_4$ are each hydrogen or lower-alkyl, or $R_3$ and $R_4$ together are divalent lower-alkylene, $-(CH_2)_m-$, where m is one of the integers 3 and 4; and Alk is lower-alkyl, which comprises reacting a compound having the Formula II (herein), where three of $R_2$, $R_2'$, $R_2''$ and $R_2'''$ are hydrogen and the fourth is lower-alkoxy, and $R_1$, $R_3$ and $R_4$ have the meanings given above, with an alkali metal lower-alkanethiol salt.

2. A process according to claim 1, for preparing 3,6-(eq),11(ax)-trimethyl-8-hydroxy-11(eq)-(6-methylmercapto-3-oxohexyl)-1,2,3,4,5,6-hexahydro-2,6-methano-3-benzazocine, which comprises reacting 3,6(eq),11(ax)-trimethyl-8-methoxy-11(eq)-(3-cyclopropyl-3-oxopropyl)-1,2,3,4,5,6-hexahydro-2,6-methano-3-benzazocine with an alkali metal methylsulfide.

3. A process according to claim 1, for preparing 3,6-(eq),11(ax)-trimethyl-8-hydroxy-11(eq)-(6-propylmercapto-3-oxohexyl)-1,2,3,4,5,6-hexahydro-2,6-methano-3-benzazocine, which comprises reacting 3,6(eq),11(ax)-trimethyl-8-methoxy-11(eq)-(3-cyclopropyl-3-oxopropyl)-1,2,3,4,5,6-hexahydro-2,6-methano-3-benzazocine with an alkali metal propylsulfide.

European Patent Office

# EUROPEAN SEARCH REPORT

0019056

Application number

EP 80 10 1417

| DOCUMENTS CONSIDERED TO BE RELEVANT | | | CLASSIFICATION OF THE APPLICATION (Int. Cl) |
|---|---|---|---|
| Category | Citation of document with indication, where appropriate, of relevant passages | Relevant to claim | |
| D(A) | US - A - 4 148 794 (T.R. LEWIS)<br>* Columns 1-3 * | 1 | C 07 D 221/26 //<br>A 61 K 31/47 |
| | | | TECHNICAL FIELDS SEARCHED (Int.Cl) |
| | | | C 07 D 221/26<br>C 07 D 221/22 |
| | | | CATEGORY OF CITED DOCUMENTS<br>X: particularly relevant<br>A: technological background<br>O: non-written disclosure<br>P. intermediate document<br>T: theory or principle underlying the invention<br>E: conflicting application<br>D: document cited in the application<br>L. citation for other reasons |
| X | The present search report has been drawn up for all claims | | &: member of the same patent family, corresponding document |

| Place of search | Date of completion of the search | Examiner |
|---|---|---|
| THE HAGUE | 18 July, 1980 | BRIGHENTI |

EPO Form 1503.1 06.78